# EUROPEAN PATENT SPECIFICATION

(11) **EP 2 384 741 B1**
(45) Date of publication and mention of the grant of the patent: **12.08.2015**
(21) Application number: 11003689.4
(22) Date of filing: 05.05.2011
(51) Int. Cl.: A61K 8/63, A61K 8/97, A61Q 17/00, A61Q 19/00, A61K 31/56, A61K 36/18, A61K 36/185

(54) **Topical product**
Topisches Produkt
Produit topique

(30) Priority: 06.05.2010 CZ 201022675 U
(43) Date of publication of application: 09.11.2011
(73) Proprietor: Sarek, Jan, 28167 Stribma Skalice (CZ); Ceské Vysoké Ucení Technické V Praze, 115 19 Praha 1 (CZ)
(72) Inventor: Sarek, Jan, 109 00 Praha 10 (CZ); Vlk, Martin, 90846 Unin (SK)
(74) Representative: Gabrielova, Marta

(56) References cited:
- WO-A1-01/72315
- WO-A2-01/10885
- WO-A2-2011/064271
- Yu-Hong Zhang ET AL: "Extraction of betulin from bark of Betula platyphylla by supercritical carbon dioxide extraction", Journal of Forestry Research, 1 January 2003 (2003-01-01), pages 202-204, XP55030313, Retrieved from the Internet: URL:http://www.springerlink.com/content/V3 1L08Q67M153J66/fulltext.pdf [retrieved on 2012-06-19]

## Description

### Field of Art

The present invention relates to a topical product destined particularly for the regeneration of damaged skin and for skin care.

### Background Art

Topical cosmetic products in the form of creams, ointments, lotions and other cosmetic formulations are commonly used for skin care and for the regeneration of damaged skin. Skin damage can be caused for example by injuries, insect bites, skin diseases (e.g. psoriasis, eczema) and the like.

Topical cosmetic products for skin regeneration contain various regeneration components with healing, greasing, antiseptic and disinfecting effects, e.g., tea tree oil, which has an antiseptic and disinfecting effect; allantoin and camomile, which have a healing effect on minor injuries and wounds; olive oil for greasing dry skin, and many others.

Creams containing these components do not always act reliably on all kinds of skin damages. For example, for the treatment of psoriasis, special healing products on an entirely different basis are used.

Until now more than 30,000 terpenoids have been isolated from natural sources, including 4,000 triterpenoids derived from more than 40 skeletons. The range of biological activities of triterpenoids (Dzubak P., Hajduch M., Vydra D., Hustova A., Kvasnica M, Biedermann D., Markova L., Urban M., Sarek J.: Nat. Prod Rep. 23, 394-411 (2006*))* is incredibly broad, it includes, e.g., antimicrobial (fusidic and helvolic acids), antifeedant (radermasinin), antimycotic (trianthenol), antiviral (derivatives of betulinic acid), antiinflamatory (derivatives of bosswelic acid, platycodin D, betulin, lupeol), antitumor effects (betulinic, bosswelic, pomolic acids), anticariogenic (oleanolic, glycyrrhetic acids), antiangiogenic, antiulcerogenic (glycyrrhetic, ursolic and oleanolic acids), antialergic (oleanolic acid), hepatoprotective (lupeol, ursolic, oleanolic acids), tonic activities (24-hydroxytormentic and bosswelic acids) and many others. Although some of the above mentioned biological activities of triperpenoids have been known for a long time, their use is known mostly in oriental or folk medicine (for example the bark of a Brazilian tree Ocotea suaveolens has been used in ingenious medicine for many years for pain relief, as a tonic and for asthma treatment), so a very small part of their pharmaceutical potential is used.

Betulin is a natural pentacyclic triterpenic alcohol (lup-20(29)-en-3β, 28-diol), which is abundant in the nature, in particular in the birch bark (*Betula sp*.), where its name also comes from. Betulin was one of the first triterpenoids to be isolated by Löwitz in 1788 by sublimation from the birch bark as a pure chemical substance *(*Simonsen J., Ross W. C. J.: The Terpenes IV, Cambridge Univ. Press, London 1957*).* Then it was obtained by extraction of the outer layers of the birch bark by ethanol. It took several decades before its structure could be exactly determined. Basically, the white colour of the birch bark is caused by the presence of betulin, which gives the birch bark its specific properties. For example, the birch bark never rots or moulds. This is due to the anti-mould and antibacterial properties of betulin. It is unknown why birches synthesise betulin and deposit it into their bark. It is probably a natural agent against pests and attacks of microorganisms. Betulin and its derivatives, however, show other interesting biological effects, e.g. antiinflamatory, antiviral, anti-HIV, hepatoprotective and others (Dzubak P., Hajduch M., Vydra D., Hustova A., Kvasnica M., Biedermann D., Markova L., Urban M., Sarek J.: Nat. Prod. Rep. 23, 394-411 (2006*)).*

Lupeol is a natural pentacyclic triterpenic alcohol (lup-20(29)-en-3β-ol), present in many herbs and fruits (e.g. apples, mango). Moreover, it accompanies betulin in the nature. Lupeol shows some chemopreventive, cytotoxic and antifeedant effects. It also shows a direct antioxidant activity and is a potential drug for diseases caused by free radicals. Other activities include the anti-angiogenic effect (Dzubak P., Hajduch M., Vydra D., Hustova A., Kvasnica M., Biedermann D., Markova L., Urban M., Sarek J.: Nat. Prod. Rep. 23, 394-411 (2006*)).*

Lupane triterpenoids are already being used in topical products in cosmetics. A Polish company Sylveco has the broadest portfolio of birch cosmetics on the market. This company offers several birch products - body cream, body lotion, hypoallergenic birch cream, birch pomade, buckthorn pomade and a dietary supplement Betuleco - all of them containing pure betulin. A wide range of applications is best shown by the scope of possible application of the hypoallergenic birch cream containing betulin as recommended by the producer - sunburns, dry hand skin, cracked heels and feet, non-pigmented and unevenly pigmented skin, abrasions, pressure sores, bedsores, cold sores, places of insect attack (mosquito, flea, fly, bee, wasp, etc.), dermatomycoses, psoriasis, acne, skin allergies, eczema, rashes, varicose veins, ischemia, bums, chemical bums (acid and caustic soda bums), frostbites. On the German market there exists a Finisa C' cream developed especially for professional cyclists against sore spots (produced by Helga Müller-Naturprodukte, Germany), which contains betulin as one of the active components. A Russian company Limonnik Co., Ltd. offers oral products and food supplements with betulin in several versions. All the mentioned products are declared to contain only betulin, respectively betulinic acid as well.

The present solution describes a topical product based on a natural basis with regeneration effects on the skin.

### Disclosure of the Invention

The object of the invention is a topical product, which contains supercritical birch bark extract as an active component. The main components of the supercritical birch bark extract are pentacyclic lupane triterpenoids betulin and lupeol, illustrated by the general formula I.

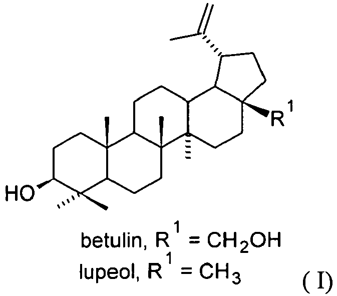

The supercritical birch bark extract is an extract prepared by the extraction of birch bark by supercritical carbon dioxide, or by supercritical carbon dioxide in combination with C₁-C₄ alcohols. This extraction is preferably performed at a temperature of from 50 to 75 °C, at a pressure of from 29 to 30 MPa for 3 to 6 hours. The usual content of betulin in the supercritical extract varies between 40 to 50 wt. % and the content of lupeol ranges around a half of the betulin content, consequently at 20 to 25 wt. %.

A good source of the birch bark on an industrial scale may be a bark waste from paper mills in Nordic countries (Finland, Sweden, Canada, Russia, Alaska), where mostly birch timber is processed. Such an average paper mill produces about 40 tons of bark waste daily (Krasutsky P.A.: Nat. Prod Rep. 23, 919-942, 2006*).* For the extraction, preferably the top layer (1-3 mm) of the birch bark (*Betula pendula* and *Betula papyrifera*) is used.

The topical product can further include auxiliary active components, preferably selected from the group comprising tea tree oil, olive oil, camomile oil, allantoin and lanolin. It may also contain natural aromatic oils, which possess inter alia also antibacterial activity (Wang H., Liu Y.: Chem. Biodivers. 7, 229-235, 2010*).*

The topical product preferably contains 500 to 1 500 mg of the birch bark supercritical extract per 1 000 g of the topical product. It can further contain 10 to 100 g of olive oil per 1 000 g of the topical product, 1 to 5 g of tea tree oil per 1000 g of the topical product, 250 to 1 000 mg of allantoin per 1 000 g of the topical product, 1 to 10 g of 20% camomile oil per 1 000 g of the topical product, 0 to 20 g, more preferably 5 to 20 g of lanolin per 1000 g of the topical product.

The lupane triterpenoids contained in the supercritical birch bark extract cure and regenerate damaged skin, olive oil moisturizes dry skin and provides it with elasticity, tea tree oil has a strong antiseptic and disinfection effect and allantoin together with camomile have a healing effect on minor injuries and wounds.

The topical product can be preferably in the form of a cream, an ointment or a lotion. With regard to the application form the topic product contains pharmaceutically or cosmetically acceptable auxiliary substances, fragrances, and suitable vehicles commonly used in the relevant application forms such as ointment and cream bases.

According to the present invention the topical product contains betulin, lupeol and other active components contained in the birch bark, in contrast to the products described in the Background Art chapter which contain betulin only, or betulinic acid. The composition of the topical product, according to the present invention, is therefore complex and respects the composition of the active components in the birch bark. The effects of the topical product according to the present invention are improved by the combination of the triterpenoid basis, olive oil and tea tree oil, camomile oil, allantoin and lanolin. The triterpenoid components bring healing and regeneration effects, the olive oil greases and softens the skin, the tea tree oil adds particularly disinfecting and antiseptic effects, the camomile has an antiinflammatory, healing and regeneration effects, allantoin has a regeneration effect and lanolin adds the necessary grease to the extent needed, so that the cream cannot be easily washed off with water.

### Brief Description of the Drawings

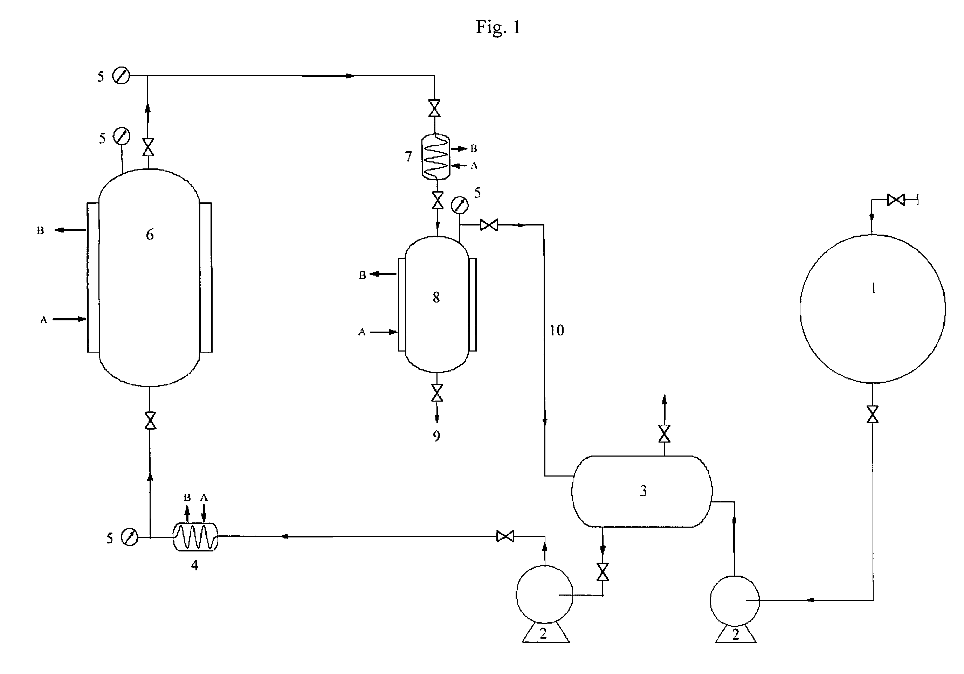

Figure 1 represents a supercritical extraction device suitable for the preparation of a supercritical extract according to Example 1. Reference signs used: 1 - liquid carbon dioxide tank, 2, 21 - compressors, 3 - carbon dioxide buffer tank, 4 - heater, 5, 51, 52, 53 - pressure gauges, 6 - supercritical extractor, 7 - cooler, 8 - separator, 9 - supercritical extract, 10 - carbon dioxide recycling (return pipeline), A - entry of a heat transfer media, B - exit of a heat transfer media.

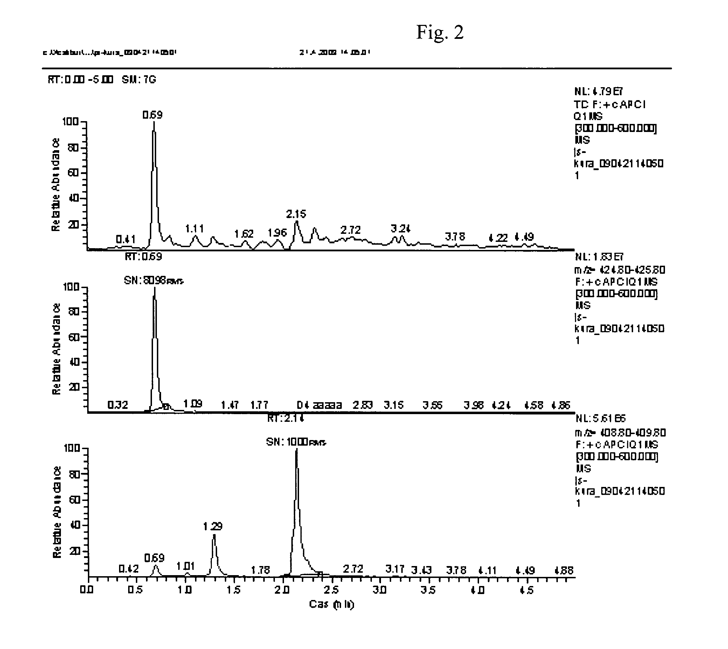

Figure 2 illustrates a HPLC chromatogram of a supercritical birch bark extract (top) and the extracted chromatograms of both main components betulin (in the middle) and lupeol (at the bottom).

### Examples of Carrying Out the Invention

### Example 1: The preparation process of a supercritical extract of birch bark

A 100 ml stainless steel extraction cartridge was filled with birch bark (21 g) and inserted into the extractor 6 of the supercritical extraction device shown in Figure 1. After closing the extractor 6, the liquid carbon dioxide was infused from the tank on the path to the compressor 2, from which it was delivered to the buffer tank 3 and then further transported by compressor 21 to the heater 4, where it was heated to a temperature of 75 °C at a pressure of 29 to 30 MPa (measured with a pressure gauge 5). The carbon dioxide, heated and compressed in this way, was then brought into the extractor 6, into which the extraction cartridge was inserted. After reaching the working pressure of 29 to 30 MPa in the extractor 6 (gauge 51) carbon dioxide is discharged through a system of valves and cooler 7 to the separator 8, where its expansion takes place (gauge 53). Meanwhile, the supercritical extract accumulates in the separator 8 and carbon dioxide is being recycled through the return pipeline 10 into the buffer tank. The cycle of carbon dioxide is thereby closed. The raw material was extracted in the supercritical extraction device as described herein by carbon dioxide at a pressure of 29 to 30 MPa, temperature of 75° C for 4 hours. At the end of the extraction a pulverized extract of ivory colour, characteristic smell in the yield of 735 mg (3.5%) was obtained in the separator, wherein the extract, according to HPLC analysis, contains 44 % by weight of betulin and 21 % by weight of lupeol.

### Example 2: Process of preparation of a topical product in the form of cream

All starting substances had a temperature of 50 to 55 ° C. Throughout the whole process of mixing the temperature of the mixture did not fall below 50 °C. Lanolin is dissolved in a mixture of hot olive oil and tea tree oil, and, after cooling to 50 °C, aromatic oils are added. Allantoin is dissolved in glycerol by heating and when being cooled, propylene glycol is added. The supercritical extract of birch bark is dissolved by heating in Solketal, then filtered and cooled to 50 °C. Ambiderman cream base is warmed up to 55 °C and then all the previously prepared solutions are mixed into it by planetary stirring. During the whole preparation process the temperature of 50 to 55 °C was maintained. First, the solution of the supercritical birch bark extract in Solketal is slowly added (within 5 minutes). Then the solution of substances in olive oil is added (within 10 to 15 minutes). Finally, the allantoin solution is added into the mixture (within 5 to 7 minutes). If necessary, the cream has to be warmed up during the mixing in a water bath in such a way that its temperature is not lower than 50 °C. After adding all the components it is stirred for another 10 minutes. Then the hot cream is filled into containers.

### Example 3: Process of preparation of a topical product in the form of lotion

All starting substances had a temperature of 50 to 55 ° C. Throughout the whole process of mixing the temperature of the mixture did not fall below 50 °C. Olive oil is mixing with tea tree oil, and, after cooling to 50 °C, aromatic oils are added. Allantoin is dissolved in glycerol by heating and when being cooled, propylene glycol and water are added. The supercritical extract of birch bark is dissolved by heating in Solketal, then filtered and cooled to 50 °C. Ambiderman cream base is warmed up to 55 °C and then all the previously prepared solutions are mixed into it by planetary stirring. During the whole preparation the temperature of 50 to 55 °C was maintained. First, the solution of the supercritical birch bark extract in Solketal is slowly added (within 5 minutes). Then the allantoin solution is added into the mixture (within 5 to 7 minutes). Finally, the solution of substances in olive oil is added (within 10 to 15 minutes). If necessary, the cream has to be warmed up during the mixing in a water bath in such a way that its temperature is not lower than 50 °C. After adding all the components it is stirred for another 10 minutes. Then the hot cream is filled into containers.

Table 1 shows the quantity of raw materials used for the production of three different batches of cream and one batch of lotion according to examples 2 and 3.

**Table 1**

| Component | Quality | CAS No. | Producer | Cream 1 g/1000 g | Cream 2 g/1000 g | Cream 3 g/1000 g | Lotion 1 g/1000 g |
|---|---|---|---|---|---|---|---|
| Ambiderman | Czech Ph. 5 | | Herbacos-Bofarma | 832.02 | 818.06 | 819.27 | 749.85 |
| Cera Lanae | Czech Ph. 5 | 8006-54-0 | Biomedica | 8.32 | 13.63 | 8.19 | 0.00 |
| Olive oil | Czech Ph. 5 | 8001-25-0 | Biomedica | 78.21 | 76.90 | 77.01 | 72.80 |
| Tea tree oil | essential | 68647-73-4 | Marta Schnaider | 3.33 | 3.27 | 3.28 | 2.91 |
| Water dist. | distilled | 7732-18-5 | EuroSarm | 0.00 | 0.00 | 0.00 | 109.21 |
| Glycerol, 85% | Ph. Eur. | 56-81-5 | Lach-Ner | 23.30 | 34.36 | 24.58 | 21.84 |
| Propylene glycol | Ph. Eur. | 57-55-6 | Merck | 19.14 | 19.09 | 18.84 | 21.84 |
| Solketal | pure | 100-79-8 | Sigma-Aldrich | 26.62 | 24.54 | 32.77 | 29.12 |
| Allantoin | pure | 97-59-6 | Sigma-Aldrich | 0.42 | 0.55 | 0.82 | 0.73 |
| Supercritical extract of the birch bark | | | see Example 1 | 1.25 | 1.36 | 1.64 | 1.46 |
| Bergamot essential oil | essential oil | | Marta Schnaider | 2.91 | 3.00 | 5.73 | 5.10 |
| Lemon oil | essential oil | | Marta Schnaider | 1.50 | 1.64 | 2.95 | 2.62 |
| Sweet violet essential oil | 10% extract in almond oil | | Marta Schnaider | 0.83 | 0.82 | 1.64 | 1.46 |
| Camomile oil | 20% in almond oil | | Marta Schnaider | 1.00 | 1.36 | 0.98 | 0.87 |
| Iris | fragrant oil | | Salus | 0.62 | 0.76 | 1.23 | 1.09 |
| Freesia | fragrant oil | | Salus | 0.33 | 0.33 | 0.66 | 0.58 |
| Blood orange | essential oil | | Marta Schnaider | 0.21 | 0.33 | 0.41 | 0.36 |
| Total weight of components | - | - | - | 1000.00 | 1000.00 | 1000.00 | 1000.00 |

Ambiderman cream base contains the following components in 100 g: paraffinum liquidum 8 g, paraffinum solidum 12 g, alcohol stearilicus 2 g, propylenglycolum 5 g, slovasol 2430 2 g, carbomera 0.5 g, trolaminum 0.6 g, methylparabenum 0.2 g, propylparabenum 0.05 g, aqua purificata 69.65 g.

The formulations described as cream 1 to cream 3 have been tested by users suffering from dry and cracked skin on hands or feet, psoriasis, and also on places after a wasp, mosquito or tick attack. A male patient who had problems with healing of small cuts and abrasions on his dried-out and cracked hands, experienced a significant improvement in healing abrasions and the skin on his hands became flexible and not so dry after a regular daily one month-long application. In case of a female patient suffering from psoriasis the affected area was palm-sized, reddish and raised above the skin above the ankle. Creams 2 or 3 were used once daily in the evening. Within two weeks of application the dried skin was replaced with a new one and the affected area blended with its surroundings. Within 1 year after the treatment the described problems did not relapse. A male patient suffering from dry and cracked skin on the soles achieved within a week normalization and healing of the skin after applying the cream 2 every other day. An allergic user who had been stabbed by wasps in the palm of his left hand felt a very strong and intensive permanent pain. After applying cream 3 he came to rest and was relieved from pain. A similar effect was also recorded when applied to painful mosquito incisions or on a place after removing a tick. There were no allergic reactions.

The formulation described as lotion 1 was tested by women having a very dry and sensitive skin, with an increased itchiness. The lotion was used always after body hygiene (shower or bath). After one month of administration, the itchiness feelings receded and the skin was less dry and sensitive. There were no allergic reactions.

### Industrial Applicability

The topical product according to the present invention has a strong antiseptic, healing and regeneration effects. The most important is the use on damaged, dry and weakened skin, particularly on the skin of limbs. The damage can be, e.g., of mechanical type, bums, chemical bums. The topical product has a positive effect on skin of people suffering from psoriasis and eczema. It brings relief when applied after an attack of insects (wasps, mosquitoes) and ticks. Further, it has a positive impact on problems with haemorrhoids. Finally, it softens and moisturizes old and hardened skin (on hands, knuckles and the heels).

## Claims

1. A topical product, **characterized in that** it contains in 1 000 g of the topical product 500 to 1500 mg of supercritical birch bark extract comprising 40 to 50 wt. % of betulin and 20 to 25 wt. % of lupeol as an active component.

2. The topical product according to claim 1, further including auxiliary active components, preferably selected from the group comprising tea tree oil, olive oil, camomile oil, allantoin and lanolin.

3. The topical product according to claim 2, containing in 1 000 g of the topical product 10 to 100 g of olive oil, 1 to 5 g of tea tree oil, 250 to 1000 mg of allantoin, 1 to 10 g of 20 % camomile oil, 0 to 20 g of lanolin.

4. The topical product according to any of claims 1 to 3, further containing pharmaceutically or cosmetically acceptable auxiliary substances, fragrances, and vehicles.

5. The topical product according to any of the preceding claims in the form of cream, ointment or body lotion.

## Patentansprüche

1. Topisches Produkt, **dadurch gekennzeichnet, dass** es in 1000 g des topischen Produktes 500 bis 1500 mg des superkritischen Extraktes aus der Birkenrinde enthaltend 40 bis 50 Gewichts-% Betulin und 20 bis 25 Gewichts-% Lupeol als Wirkstoff enthält.

2. Topisches Produkt nach Anspruch 1, weiter enthaltend Hilfswirkstoffe, vorzugsweise ausgewählt aus der Gruppe enthaltend Tea-Tree-Öl, Olivenöl, Kamillenöl, Allantoin und Lanolin.

3. Topisches Produkt nach Anspruch 2, enthaltend in 1000 g des topischen Produktes 10 bis 100 g Olivenöl, 1 bis 5 g Tea-Tree-Öl, 250 bis 1000 mg Allantoin, 1 bis 10 g 20%-Kamillenöl, 0 bis 20 g Lanolin.

4. Topisches Produkt nach einem der Ansprüche 1 bis 3, weiter enthaltend pharmazeutisch oder kosmetisch annehmbare Hilfsstoffe, Duftkomponenten und Trägersubstanzen.

5. Topisches Produkt nach einem der vorherigen Ansprüche in Form von Creme, Salbe oder Körpermilch.

## Revendications

1. Le produit topique, caractérisé en qu'il contient dans 1000 g de produit topique 500 jusqu'à 1500 mg de l'extrait supercritique d'écorce de bouleau contenant 40 jusqu'à 50 pour cent de poids de bétuline et 20 jusqu'à 25 pour cent de poids de lupéol en tant que produit efficace.

2. Le produit topique selon la revendication 1, contenant des produits auxiliaires efficaces, de préférence choisis du groupe contenant de l'huile d'arbre à thé, de l'huile d'olive, de l'huile de camomille, d'allantoïne et de lanoline.

3. Le produit topique selon la revendication 2, contenant dans 1000 g de produit topique 10 jusqu'à 100 g de l'huile d'olive, 1 jusqu'à 5 g de l'huile d'arbre à thé, 250 jusqu'à 1000 mg d'allantoïne, 1 jusqu'à 10 g 20 % de l'huile de camomille, 0 jusqu'à 20 g de lanoline.

4. Le produit topique selon les revendications 1 jusqu'à 3, contenant des matières auxiliaires pharmaceutiquement ou cosmétiquement acceptables, des composants aromatiques et des véhicules.

5. Le produit topique selon une quelconque revendication précédente, étant en forme de crème, de pommade ou de lait corporel.
